Europäisches Patentamt

European Patent Office

Office européen des brevets

(11) Veröffentlichungsnummer: **0 479 014 A1**

# (12) EUROPÄISCHE PATENTANMELDUNG

(21) Anmeldenummer: **91115633.9**

(22) Anmeldetag: **14.09.91**

(51) Int. Cl.5: **A61F 5/02**, A61F 5/34

---

(30) Priorität: **01.10.90 DE 9013695 U**

(43) Veröffentlichungstag der Anmeldung:
**08.04.92 Patentblatt 92/15**

(84) Benannte Vertragsstaaten:
**AT CH DE FR IT LI NL SE**

(71) Anmelder: **FERD. HAUBER GmbH & CO. KG**
**Sigmaringer Strasse 14-16**

W-7440 Nürtingen(DE)

(72) Erfinder: **Hildebrandt, Hans-Dietrich, Dr.**
**Hohlesteinweg 16**
**W-3501 Ahnatal(DE)**

(74) Vertreter: **Becker, Maria, Dipl.-Phys.**
**Auf dem Haigst 29**
**W-7000 Stuttgart 70(DE)**

---

(54) **Aufblasbares Luftkissen für orthopädische Hilfsgegenstände, wie Stützbandagen.**

(57) Zur Therapie von Lumbalgie und anderen Beschwerden vorwiegend im Rückenbereich ist ein aufblasbares Luftkissen, insbesondere für Kreuzstützbandagen, vorgesehen, welches aus mindestens einer schweissbaren Materialbahn hergestellt ist und die miteinander verbundenen Luftkammern durch zueinander senkrecht verlaufende, unterbrochene Schweissnähte gebildet sind.

Fig. 2

EP 0 479 014 A1

Die Erfindung betrifft ein aufblasbares Luftkissen für orthopädische Hilfsgegenstände, wie Stützbandagen, Gürtel und Korsetts, mit einer Vielzahl von untereinander verbundenen und über einen verschliessbaren Abschlußstutzen aufblasbaren Luftkammern.

Derartige Luftkissen sind zur Verwendung im orthopädischen Bereich, insbesondere für Kreuzstützbandagen, bekannt. Sie werden zur Behandlung von chronischer lokaler oder pseudoradikulärer Lumbalgie bei Degenerationen und muskulärer Insuffizienz angewendet.

Aus der DE-A 27 09 369 ist ein orthopädisches Korsett zur Korrektur von Verformungen der Wirbelsäule bekannt mit Versteifungsgerüsten, die aus mehreren in Verbund stehenden und über einen verschliessbaren Stutzen aufblasbaren Rohren bestehen. Dabei erstrecken sich die Rohre über die gesamte Rückenpartie und ergeben ein ziemlich starres, unbequemes Rückengerüst.

Man kennt auch einen sogenannten "Halo-Fixateur" (DBGM 86 10 887) mit einer der Brustpartie anzulegenden Weste, wobei die Weste innenseitig mit wenigstens einem flexiblen Aufblaskörper versehen ist. Es handelt sich hierbei um ein einziges Luftkissen mit glatten Aussenwänden.

Der Erfindung liegt die Aufgabe zugrunde, ein einfach herstellbares aufblasbares Luftkissen für orthopädische Zwecke, insbesondere für Stützbandagen, zu schaffen, welches eine optimale therapeutische Funktion hat und dennoch angenehm zu tragen ist. Diese Aufgabe wird dadurch gelöst, dass das Luftkissen aus mindestens einer schweissbaren Materialbahn gebildet ist und die miteinander verbundenen Luftkammern durch zueinander senkrecht verlaufende unterbrochene Schweissnähte gebildet sind. Dieses Luftkissen besteht somit aus mehreren parallel zueinander liegenden Luftkammern, deren Füllungszustand durch Aufpumpen mittels eines Anschlußstutzens individuell der jeweiligen Anatomie des Benutzers angepasst werden kann. Die Luftkammern gewährleisten eine gleichmässige Dicke des Kissens und damit eine gleichmässige Flächenpressung am Körper. Es ergibt sich damit eine sehr gute Körperformschlüssigkeit, die im LWS-Bereich des Trägers einen guten Halt gewährleistet und gleichzeitig aber auch eine massierende Wirkung zur Lockerung und Hyperaemisierung der Muskulatur bewirkt. Die Anpassung der Luftkissenpelotte an die Körperform wird über entsprechende Verschiebung der Luftmengen in den untereinander verbundenen Luftkissen erreicht. Dadurch ergibt sich ein optimaler Tragekomfort, z.B. beim längeren Anlehnen beim Autofahren usw.

Zweckmässigerweise kann das Luftkissen aus einer einfach umgefalteten, abgeschweissten Kunststoffolie gebildet sein, wobei der Anschlußstutzen an der durch das Umfalten gebildeten Kissenkante angeschweisst ist.

Eine besonders angenehm zu tragende Form wird dadurch erreicht, dass die der umgefalteten Kissenkante gegenüberliegende Kissenkante abgerundet ist.

Ausführungsbeispiele des Gegenstandes der Erfindung sind in der Zeichnung dargestellt, darin zeigen:

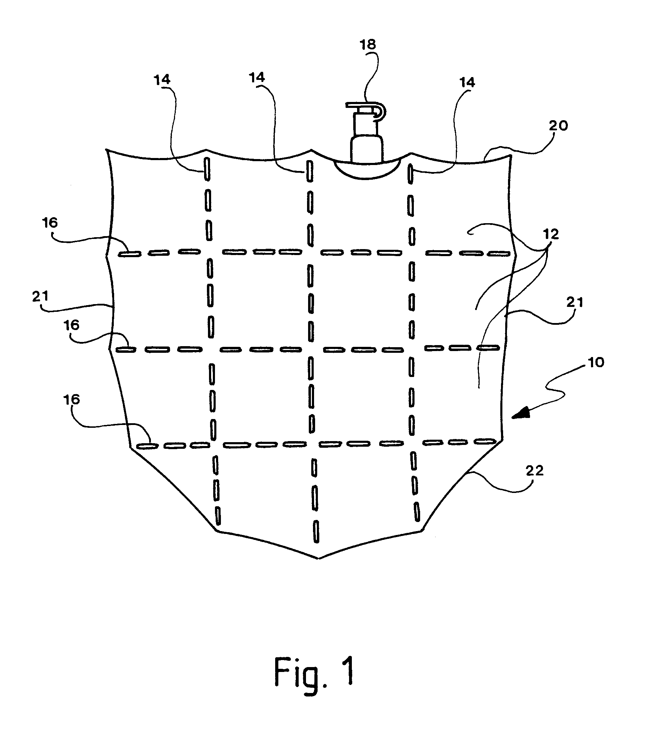

Fig. 1    eine Draufsicht auf ein aufblasbares Luftkissen nach der Erfindung:

Fig. 2    eine Draufsicht auf die Innenseite einer Kreuzstützbandage, in welcher das Luftkissen angebracht ist.

Das in der Zeichnung dargestellte aufblasbare Luftkissen 10 ist bei diesem Ausführungsbeispiel wappenförmig ausgebildet. Es besteht aus einer schweissbaren Materialbahn, vorzugsweise aus Kunststoff, die an der oberen Querseite 20 umgefaltet ist. Sie ist an ihren Rändern 20, 21 und 22 abgeschweisst und weist in ihrem Innern zueinander senkrecht verlaufende vertikale und horizontale unterbrochene Schweissnähte 14 und 16 auf. Durch die Unterbrechung der Schweissnähte ist gewährleistet, dass die Luft in den einzelnen Kammern 12 miteinander in Verbindung steht, so dass sich ein gleichmässiger Luftdruck und damit auch eine gleichmässige Dicke des Kissens bilden kann. Es ist ferner ersichtlich, dass die Luftkammern 12 gleichzeitig eine noppenartige Wirkung haben, wodurch eine gewünschte angenehme Massagewirkung erzielt wird.

An der durch das Umfalten gebildeten oberen Kissenkante 20 ist ein Anschlußstutzen 18 angeschweisst, mittels dem das Kissen auf den gewünschten Füllungszustand aufgepumpt wird.

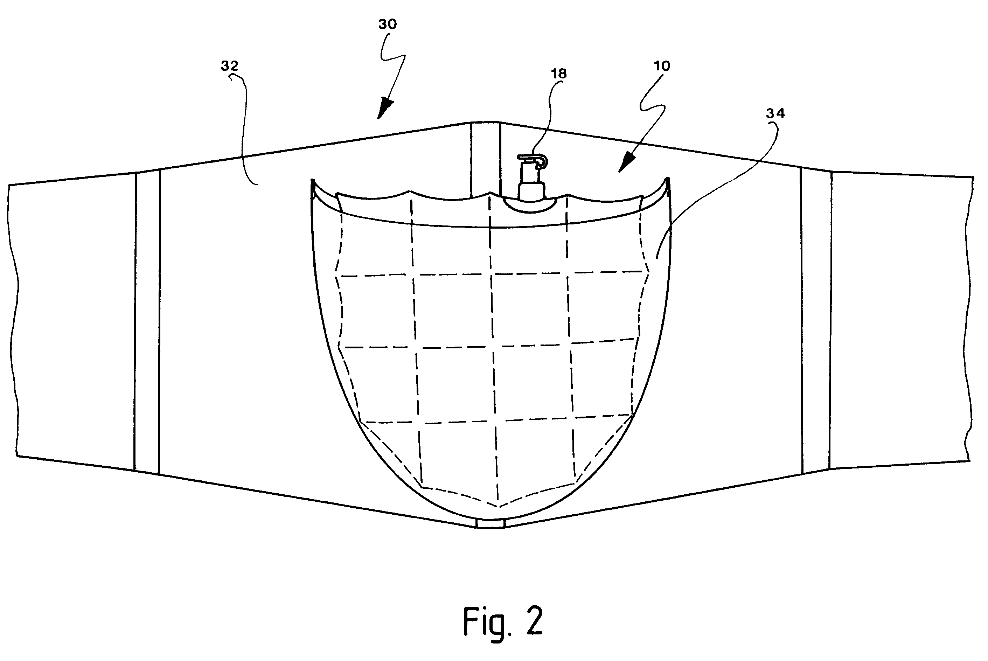

Die Figur 2 zeigt eine insgesamt mit 30 bezeichnete Kreuzstützbandage aus einem elastischen Band 32, welches eine Tasche 34 zur Aufnahme des Kissens 10 aufweist.

Diese Pelottentasche kann aus hautfreundlichem Material bestehen. Es ist ersichtlich, dass der Stutzen 18 leicht zugänglich und einfach zu betätigen ist.

Die abgerundeten Seiten des Kissens geben keine Druckstellen auf der Haut, vielmehr passt sich das Kissen der jeweiligen Körperanatomie hervorragend an und ergibt einen guten Flächendruck am Körper.

**Patentansprüche**

1.   Aufblasbares Luftkissen für orthopädische Hilfsgegenstände, wie Stützbandagen, Gürtel und Korsetts, mit einer Vielzahl von untereinander verbundenen und über einen verschliessbaren Abschlußstutzen aufblasbaren Luftkammern,

**dadurch gekennzeichnet,**

dass das Luftkissen (10) aus mindestens einer schweissbaren Materialbahn gebildet ist und die miteinander verbundenen Luftkammern (12) durch zueinander senkrecht verlaufende, unterbrochene Schweissnähte (14, 16) gebildet sind.

2. Luftkissen nach Anspruch 1, dadurch gekennzeichnet, dass es aus einer einfach umgefalteten, abgeschweissten Kunststofffolie gebildet ist.

3. Luftkissen nach Anspruch 1 oder 2, dadurch gekennzeichnet, dass der Anschlußstutzen (18) an der durch das Umfalten gebildeten Kissenkante angeschweisst ist.

4. Luftkissen nach einem der vorhergehenden Ansprüche, dadurch gekennzeichnet, dass die der umgefalteten Kissenkante (20) gegenüberliegende Kissenkante (22) abgerundet ist.

Fig. 1

Fig. 2

EP 0 479 014 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| --- | --- | --- | --- |
| Y | DE-A-1 491 199 (NICOLL) <br> * Ansprüche 1,13 * * <br> – – – | 1,2,4 | A 61 F 5/02 <br> A 61 F 5/34 |
| Y | GB-A-822 684 (U.S. RUBBER COMP.) <br> * Ansprüche 1,2; Abbildung 1 * * <br> – – – | 1,2,4 | |
| A | US-A-4 682 588 (CURLEE) <br> * Zusammenfassung; Abbildungen * * <br> – – – | 1 | |
| A | EP-A-0 238 183 (CURLEE) <br> * Seite 8, Absatz 2; Abbildungen 1,2 * * <br> – – – | 4 | |
| A | GB-A-2 094 631 (SPENCER LTD) <br> – – – – – | - | |

|  | RECHERCHIERTE SACHGEBIETE (Int. Cl.5) |
| --- | --- |
| | A 61 F <br> A 61 G <br> A 47 C |

**Der vorliegende Recherchenbericht wurde für alle Patentansprüche erstellt**

| Recherchenort | Abschlußdatum der Recherche | Prüfer |
| --- | --- | --- |
| Den Haag | 12 Dezember 91 | VILLENEUVE J-M.R.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument